# EUROPEAN PATENT APPLICATION

(11) **EP 4 389 729 A1**
(43) Date of publication of application: **26.06.2024**
(21) Application number: 22873091.7
(22) Date of filing: 28.07.2022
(51) Int. Cl.: C07C 17/04, C07C 17/26, C07C 19/10, C07C 19/14, C07C 17/23, C07C 17/35, C07C 21/18

(54) **METHOD FOR PRODUCING 1,4-DIBROMO-2,3-DICHLOROHEXAFLUOROBUTANE AND METHOD FOR PRODUCING HEXAFLUORO-1,3-BUTADIENE USING SAME**

(30) Priority: 23.09.2021 KR 20210125650
(71) Applicant: Jin Sung Eng Co., Ltd., Asan-si, Chungcheongnam-do 31415 (KR)
(72) Inventor: JANG, Jun Jae, Suwon-si, Gyeonggi-do 16509 (KR); KIM, Sang Ik, Seongnam-si, Gyeonggi-do 13485 (KR); CHO, Wook Jae, Ulsan 44452 (KR); PARK, Hong Lual, Yangpyeong-gun, Gyeonggi-do 12507 (KR)
(74) Representative: Mammel und Maser Patentanwälte PartG mbB
(86) International application number: PCT/KR2022/011140
(87) International publication number: WO 2023/048385

(57) **Abstract**

Disclosed is a method for producing 1,4-dibromo-2,3-dichlorohexafluorobutane (Br-CF₂-CFCl-CFCl-CF₂-Br), in which, through the photoreaction of 1,2-dibromo-1-chlorotrifluoroethane (Br-CF2-CFCl-Br) with a CTFE (CF2=CFCl) gas mixed with a diluent gas, 1,4-dibromo-2,3-dichlorohexafluorobutane is produced. According to an embodiment of the present disclosure, an intermediate for producing hexafluoro-1,3-butadiene (C₄F₆) can be produced with high production yield.

## Description

### Technical Field

This application claims the benefit of priority from Korean Patent Application No. 2021-0125650, filed September 23, 2021, and all disclosures in that Korean patent application are hereby incorporated by reference as part of this specification.

The present disclosure relates to a method for preparing 1,4-dibromo-2,3-dichlorohexafluorobutane and a method for preparing hexafluoro-1,3-butadiene using the same.

### Background Art

Hexafluoro-1,3-butadiene (C₄F₆) is a specialty gas used as an etchant in a semiconductor manufacturing process, and its demand is increasing as the integration degree of semiconductor devices increases.

Various techniques for preparing C₄F₆ are disclosed in some related art documents. The techniques disclosed in the related art documents include a method of preparing X-CF₂-CFY-CFY-CF₂-X (X=Cl, Br, I, Y=Cl, Br, I, F) as an intermediate to facilitate the preparation of C₄F₆. Since C₄F₆ can be readily prepared from these intermediates, the manufacturing cost of C₄F₆ varies greatly depending on the intermediate preparation method.

The materials described below may be used as intermediates.

① I-CF₂-CF₂-CF₂-CF₂-I

② Br-CF₂-CF₂-CF₂-CF₂-Br

③ Cl-CF₂-CFCl-CFCl-CF₂-Cl

④ Br-CF₂-CFCl-CFCl-CF₂-Br

Among these intermediates, ① and ② may be prepared by Reaction Formulas (1) and (2) with a starting material of tetrafluoroethylene (TFT, CF₂=CF₂).

(1) CF₂=CF₂+ X₂ (X=Br, I) → X-CF₂-CF₂-X

(2) X-CF₂-CF₂-X + CF₂=CF₂ → X-CF₂-CF₂-CF₂-CF₂-X

The problem with the above preparation method is that it uses TFE as a starting material. In order to prepare TFE, a thermal decomposition process of R-22 (CHClF₂) is required, and it is expensive to build facilities for preparing TFE. In addition, the strong binding of fluorine atoms (F) requires a large amount of energy for Reaction Formula 2, and various byproducts are produced as a result.

Among the intermediates, ③ and ④ have the advantage that they can be prepared using chlorotrifluoro ethylene (CTFE, CF₂=CFCl), which is relatively easier to transport and purchase than TFE, as a starting material. The intermediate ③ can be prepared by Reaction Formulas (3) to (6).

(3) CF₂=CFCl + I-Cl → Cl-(CF₂-CFCl)-I

(4) 2 Cl-(CF₂-CFCl)-I + Zn → CF₂Cl-CFCl-CFCl-CF₂Cl + ZnI₂

(5) ZnI₂ → Zn + I₂

(6) I₂+ Cl₂ → 2 I-Cl

The reason for using I-Cl instead of iodine (I₂) in Reaction Formula (3) is that the I atom of I-Cl reacts with the Cl atom of CTFE (CF₂=CFCl), and the Cl atom of I-Cl reacts with the F atom of CTFE (CF₂=CFCl), and afterwards, the deiodination reaction of Reaction Formula (4) occurs to produce the intermediate ③ (CF₂Cl-CFCl-CFCl-CF₂Cl).

However, when I-Cl is added to CTFE (CF₂=CFCl), I-(CF₂-Cl)-Cl is produced as a byproduct. Therefore, instead of the intermediate (3), Cl-CF₂-CFCl-CF₂-CFCl-Cl is produced in a certain proportion, resulting in a lower yield of the intermediate (3). In addition, due to the high price of iodine (I₂) required for the production of I-Cl, it is necessary to produce iodine through a regeneration process corresponding to Reaction Formula (5), which makes the overall reaction process complicated.

An alternative to the preparation of the intermediate ③ is using Reaction Formulas (7) and (8).

(7) CH₂=CH-CH=CH₂+ 2Cl₂ → CH₂Cl-CHCl-CHCl-CH₂Cl

(8) CH₂Cl-CHCl-CHCl-CH₂Cl + 6F₂ → Cl-CF₂-CFCl-CFCl-CF₂-Cl + 6HF

However, these manufacturing processes use highly reactive fluorine (F₂), the processes have difficulty in controlling byproduct formation during the reactions. Moreover, a fluoride (F₂) electrolyzer is required to produce F₂, which increases the production cost.

### Disclosure

### Technical Problem

The present disclosure has been made to solve the above-described problems occurring in the related art, and aims to provide a method of preparing an intermediate enabling the preparation of C₄F₆ in a simple, safe, and inexpensive way and a method of preparing C₄F₆ using the intermediate.

The present disclosure provides an intermediate preparation method capable of improving an intermediate production yield by suppressing the generation of high BP materials and isomers during the intermediate production process and provides a method of preparing C₄F₆ using the same method.

The objectives to be achieved by the present disclosure are not limited to the ones mentioned above, and other objectives not mentioned above can be clearly understood by those skilled in the art from the following description.

### Technical Solution

A method of preparing 1,4-dibromo-2,3-dichlorohexafluorobutane (Br-CF₂-CFCl-CFCl-CF₂-Br), according to an embodiment of the present disclosure, is characterized in that a 1,2-dibromo-1-chlorotrifluoroethane (Br-CF₂-CFCl-Br) solution diluted with a solvent is reacted with CTFE (CF₂=CFCl) mixed with a diluent gas by light (UV) initiation to produce 1,4-dibromo-2,3-dichlorohexafluorobutane.

The solvent may include methylene chloride (MC, CH₂Cl₂), and the content of the solvent in the 1,2-dibromo-1-chlorotrifluoroethane (Br-CF₂-CFCl-Br) solution may be 90 mol% or less.

The diluent gas may include an inert gas.

Alternatively, the diluent gas may include hydrofluorocarbon (HFC) or perfluorocarbon (PFC) gas.

Alternatively, the diluent gas may include at least one inert gas and at least one gas selected from hydrofluorocarbon (HFC) and perfluorocarbon (PFC) gases.

The content of the CTFE gas in the total gas including the diluent gas and the CTFE gas may be 50 mol% or less.

In addition, the method of preparing 1,4-dibromo-2,3-dichlorohexafluorobutane according to one embodiment of the present disclosure may further include reacting CTFE (CF₂=CFCl) with bromine (Br₂) to prepare 1,2-dibromo-1-chlorotrifluoroethane (Br-CF₂-CFCl-Br).

A method of preparing hexafluoro-1,3-butadiene (C₄F₆) using 1,4-dibromo-2,3-dichlorohexafluorobutane (Br-CF₂-CFCl-CFCl-CF₂-Br) as an intermediate, according to one embodiment of the present disclosure, includes: a photoreaction step of preparing 1,4-dibromo-2,3-dichlorohexafluorobutan by initially reacting 1,2-dibromo-1-chlorotrifluoroethane (Br-CF₂-CFCl-Br) solution diluted with a solvent with a CTFE (CF₂=CFCl) gas mixed with a diluent gas by light (UV) initiation; and a dehalogenation step of removing halogen atoms, except fluorine atoms, from the prepared 1,4-dibromo-2,3-dichlorohexafluorobutane.

In this case, the dehalogenation step may be carried out in the presence of zinc (Zn) and isopropyl alcohol.

In addition, the method may further include the step of reacting CTFE (CF₂=CFCl) with bromine (Br₂) to produce 1,2-dibromo-1-chlorotrifluoroethane (Br-CF₂-CFCl-Br) prior to the photoreaction step.

### Advantageous Effects

According to one embodiment of the present disclosure, CTFE, which is relatively easy to transport and purchase, is used as a starting material to prepare 1,2-dibromo-1-chlorotrifluoroethane, which is a reaction raw material, then the 1,2-dibromo-1-chlorotrifluoroethane is reacted with CTFE to prepare 1,4-dibromo-2,3-dichlorohexafluorobutane, which is an intermediate, and C₄F₆ is then produced from the intermediate. Therefore, it is possible to produce intermediates for C₄F₆, and C₄F₆ at low cost in a simple and safe way.

In addition, according to one embodiment of the present disclosure, after diluting the reaction raw material, 1,2-dibromo-1-chlorotrifluoroethane, with a solvent such as methylene chloride (MC, CH₂Cl₂), when reacting the diluted solution with CTFE by light (UV) initiation, a diluent gas containing an inert gas is used. Therefore, the generation of high BP materials is suppressed during the intermediate preparation process, and the intermediate production yield can be improved.

In addition, according to one embodiment of the present disclosure, after diluting the reaction raw material, 1,2-dibromo-1-chlorotrifluoroethane, with a solvent such as methylene chloride (MC, CH₂Cl₂), when reacting the diluted solution with CTFE by light (UV) initiation, a diluent gas containing HFC or PFC gas is used. Therefore, the generation of isomers is suppressed during the intermediate preparation process, and the intermediate production yield can be improved.

The effects and advantages are not limited to the ones mentioned above, and other effects and advantages which are not mentioned above can be clearly understood by those skilled in the art from the following description.

### Description of Drawings

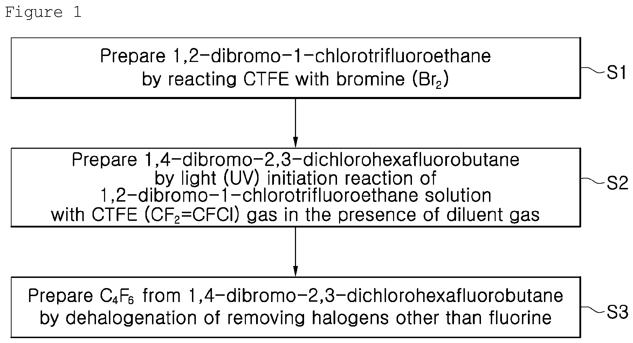

FIG. 1 is a flow diagram of a method of preparing 1,4-dibromo-2,3-dichlorohexafluorobutane (Br-CF₂-CFCl-CFCl-CF₂-Br) as an intermediate and C₄F₆ according to one embodiment of the present disclosure.

### Best Mode

Hereinafter the present disclosure will be described in detail. The following description includes specific embodiments, but the present disclosure is not intended to be limited or restricted by the embodiments described. In relation to describing the present disclosure, when the detailed description of the relevant known technology is determined to unnecessarily obscure the gist of the present disclosure, the detailed description may be omitted.

The present disclosure provides a method for preparing 1,4-dibromo-2, 3-dichlorohexafluorobutane (Br-CF₂-CFCl-CFCl-CF₂-Br) as an intermediate for the preparation of C₄F₆, and provides a method for preparing C₄F₆ using the intermediate.

A flow diagram of a method of the preparation of 1,4-dibromo-2,3-dichlorohexafluorobutane as an intermediate and C₄F₆ according to an embodiment of the present disclosure is illustrated in FIG. 1.

Referring to FIG. 1, a method of preparing 1,4-dibromo-2,3-dichlorohexafluorobutane (Br-CF₂-CFCl-CFCl-CF₂-Br) as an intermediate according to an embodiment of the present disclosure includes: a process (step S1) of reacting CTFE (CF₂=CFCl) with bromine (Br₂) to prepare 1,2-dibromo-1-chlorotrifluoroethane (Br-(CF₂-CFCl) -Br) (step S1) ; and a process (step S2) of preparing 1,4-dibromo-2,3-dichlorohexafluorobutane as an intermediate by an UV-initiated reaction of a solution of 1,2-dibromo-1-chlorotrifluoroethane with CTFE in the presence of a diluent gas.

In addition, the method of preparing C₄F₆ according to one embodiment of the present disclosure may include a dehalogenation process (step S3) to detach halogens, except fluorine, from the 1,4-dibromo-2,3-dichlorohexafluorobutane intermediate. Here, the dehalogenation reaction can be carried out by using a metal such as zinc (Zn) in the presence of a solvent such as isopropyl alcohol.

Hereinafter, the method will be described step by step in detail.

### <Preparation of 1,2-dibromo-1-chlorotrifluoroethane (Step S1)>

Step S1 is the step of reacting CTFE (CF₂=CFCl) with bromine (Br₂) to prepare 1,2-dibromo-1-chlorotrifluoroethane (Br-(CF₂-CFCl)-Br), and the step may proceed according to Reaction Formula (9).

(9) CF₂=CFCl + Br₂ → Br-(CF₂-CFCl)-Br

The process of Reaction Formula (9) may be carried out by introducing CTFE in a gaseous state and bromine (Br₂) in a liquid state into a Teflon tube immersed in an external temperature-controlled bath at the same time. CTFE gas comes into contact with liquid bromine while passing through the Teflon tube, thereby producing 1,2-dibromo-1-chlorotrifluoroethane. Excess CTFE that has passed through the tube can be recovered at the end of the tube and reused.

### <Preparation of 1,4-dibromo-2,3-dichlorohexafluorobutane (Step S2)>

Step S2 is the step of reacting CTFE with a 1,2-dibromo-1-chlorotrifluoroethane solution which is prepared by mixing the 1,2-dibromo-1-chlorotrifluoroethane prepared in step S1 with a solvent, to prepare 1,4-dibromo-2,3-dichlorohexafluorobutane. Step S2 which may proceed according to Reaction Formula (10).

(10) Br-(CF₂-CFCl)-Br + CF₂=CFCl → Br-CF₂-CFCl-CFCl-CF₂-Br

Step S2 can then proceed by light (UV) initiation in the presence of a dilute gas.

In addition, the solvent used to prepare the 1,2-dibromo-1-chlorotrifluoroethane solution is not limited to any particular solvent, but can be any solvent capable of stabilizing light (UV) initiated radicals. Preferably, the solvent may be methylene chloride (MC, CH₂Cl₂).

The diluent gas used in step S2 may be an inert gas such as nitrogen, helium, argon, etc. Alternatively, the diluent gas may be a hydrofluorocarbon (HFC) gas such as R-23 (CHF₃), R32 (CH₂F₂), R-41 (CH₃F), R-134a (CF₃CH₂F), R-125 (CF₃CHF₂), or a perfluorocarbon (PFC) gas such as CF₄, C₂F₆, or C₃F₈. Alternatively, a mixture of one inert gas and either one of HFC and PFC gas may be used. Preferably, a mixture of at least one inert gas and at least one gas selected from HFC and PFC gases may be used.

The process of Reaction Formula (10) can be carried out by filling a photoreactor equipped with a radical initiation lamp with a solution of 1,2-dibromo-1-chlorotrifluoroethane as a reaction raw material, performing a purging process composed of vacuuming and inert gas feeding several times, and then supplying diluent gas and CTFE gas in a certain ratio to the photoreactor. The diluent gas and the CTFE gas can be circulated through the photoreactor by using a compressor.

The 1,4-dibromo-2,3-dichlorohexafluorobutane as an intermediate produced in the photoreactor and the 1,2-dibromo-1-chlorotrifluoroethane solution as a reaction raw material can be transferred to a separation column. The separation column may include a first separation column and a second separation column.

A mixed solution of the 1,4-dibromo-2,3-dichlorohexafluorobutane intermediate produced in the photoreactor and the 1,2-dibromo-1-chlorotrifluoroethane reaction raw material solution produced in the photoreactor may be transferred from the photoreactor to the first separation column. The reaction raw material solution separated at the top of the first separation column can be circulated back to the photoreactor for further photoreaction.

At the bottom of the first separation column, a mixture of the intermediate, which is a photoreaction product, byproducts (high BP materials), and the reaction raw materials may be collected. When the concentration of the intermediate in the mixture exceeds a certain level, the mixture may be transferred to the second separation column for purification of the intermediate. In the second separation column, the 1,2-dibromo-1-chlorotrifluoroethane solution, which is a low boiler and a reaction raw material, is first separated and re-fed to the photoreactor, and the intermediate, 1,4-dibromo-2,3-dichlorohexafluorobutane, is then recovered by distillation. Other BP materials can be separately collected in the lower part of the second separation column after the distillation and recovery of the intermediate and are then disposed of.

The solvent used in the reaction and the inert gas in the diluent gas serve to inhibit the generation higher BP materials than 1,4-dibromo-2,3-dichlorohexafluorobutane. Here, the high BP materials may be Br-(CF₂-CFCl)ₙ-Br (n is an integer greater than or equal to 3). More specifically, selectivity for products during the reaction may vary depending on the concentration of the CTFE gas dissolved in the reaction raw material, i.e., 1,2-dibromo-1-chlorotrifluoroethane. The higher the CTFE gas concentration, the more likely that high boiling materials can be generated, and the lower the CTFE gas concentration, the more likely that the generation of high boiling materials will be suppressed and 1,4-dibromo-2,3-dichlorohexafluorobutane will be easily generated. The proportion of 1,2-dibromo-1-chlorotrifluoroethane in the 1,2-dibromo-1-chlorotrifluoroethane solution may range from 10 to 100 mol%, the proportion being chosen taking into account reactivity and selectivity.

The ratio of the CTFE gas to the diluent gas may be adjusted such that the CTFE gas content is in a range of 1 to 50 mol%, preferably a range of 1 to 25 mol%, based on the mole numbers of the entire gas including the diluent gas and the CTFE gas.

In addition, the HFC or PFC gases in the diluent gas can inhibit the formation of 1,4-dibromo-1,3-dichlorohexafluorobutane (Br-CF₂-CFCl-CF₂-CFCl-Br), which is an isomer of 1,4-dibromo-2,3-dichlorohexafluorobutane (Br-CF₂-CFCl-CFCl-CF₂-Br). That is, the yield of the intermediate, i.e., 1,4-dibromo-2,3-dichlorohexafluorobutane, can be improved by performing the reaction in the presence of a dilute gas.

When a diluent gas containing HFC or PFC gases, which are insensitive to light (UV), are used in the reaction, the generation of radicals (Br • + • CF₂-CFClBr) that contribute to the formation of isomers can be suppressed, and radicals (Br-CF₂-CFCl • + • Br) that favor the formation of the intermediate, 1,4-dibromo-2,3-dichlorohexafluorobutane, can be stably generated. Therefore, the use of a diluent gas containing HFC or PFC gases during the photoreaction can suppress the formation of isomers and improve the yield of the intermediate, 1,4-dibromo-2,3-dichlorohexafluorobutane.

### <Preparation of hexafluoro-1,3-butadiene (C₄F₆) (Step S3)>

Step S3 is a step of producing C₄F₆ by removing halogen atoms (Br and Cl) other than fluorine atoms from the 1,4-dibromo-2,3-dichlorohexafluorobutane intermediate prepared in step S2. To this end, zinc (Zn) as a metal and isopropyl alcohol as a solvent may be used. Step S3 can proceed by Reaction Formula (11).

(11) Br-CF₂-CFCl-CFCl-CF₂-Br + 2Zn/i-PrOH → C₄F₆+ 2ZnClBr

As described above, according to one embodiment of the present disclosure, CTFE, which is relatively easy to transport and obtain, is used as a starting material to prepare 1,4-dibromo-2,3-dichlorohexafluorobutane, which is an intermediate, and C₄F₆ is prepared from the intermediate. Therefore, it is possible to produce the intermediate and C₄F₆ at relatively low cost.

In addition, according to one embodiment of the present disclosure, when reacting 1,2-dibromo-1-chlorotrifluoroethane, which is a reaction raw material, with CTFE, a solvent is used to dilute the 1,2-dibromo-1-chlorotrifluoroethane, and a diluent gas including an inert gas is used to suppress the generation of high BP materials during the intermediate generation process, thereby improving the intermediate production yield.

In addition, according to one embodiment of the present disclosure, when reacting 1,2-dibromo-1-chlorotrifluoroethane, which is a reaction raw material, with CTFE, a diluent gas including HFC or PFC gas is used, thereby suppressing the generation of isomers during the intermediate generation process, and improving the intermediate production yield.

The effectiveness of the present disclosure depending on the type of diluent gas and the ratio of the diluent gas and the CTFE gas in step S2 according to Reaction Formula (10) will be described below through specific experimental examples.

### 1. Preparation of 1,4-dibromo-2,3-dichlorohexafluorobutane intermediate

A 34-L photoreactor equipped with a radical lamp was filled with 26 L of a diluted solution in which 1,2-dibromo-1-chlorotrifluoroethane as a reaction raw material and methylene chloride (MC, CH₂Cl₂) as a solvent are mixed in varying proportions, a vacuum was created to remove the air in the reactor before supplying a diluent gas and CTFE gas to the reactor. These gases were circulated through the photoreactor under constant pressure, with the lamp operating to initiate a photoreaction, and gas chromatography (GC) analysis was performed on products sampled from the bottom of the photoreactor at hourly intervals. During the reaction, the CTFE gas was continuously injected through a regulator to maintain a constant pressure.

First, the effect of the solvent content in the 1,2-dibromo-1-chlorotrifluoroethane solution was investigated by varying the ratio of 1,2-dibromo-1-chlorotrifluoroethane to solvent under 100 mol% CTFE (Comparative Examples 1 to 4). Methylene chloride (CH₂Cl₂) was used as the solvent, but similar results were obtained with other solvents such as chloroform (CHCl₃) and Carbon tetrachloride (CCl₄).

To investigate the effect of the ratio of the diluent gas to the CTFE gas, nitrogen (N₂) was used as the diluent gas under the solution condition of Comparative Example 3, and the content of the CTFE gas in the total gas (sum of the nitrogen gas and the CTFE gas) was varied from 2 to 50 mol% (Examples 1 to 4).

In addition, to investigate the effect of the number of lamps for light (UV) initiation in the reaction, the number of operating lamps was increased to three (Example 5) and seven (Example 6) before proceeding with the reaction.

In addition, to investigate the effect of the diluent gas containing HFC or PFC gas, the reaction was carried out while varying the ratio of nitrogen gas to HFC or PFC gas while maintaining the CTFE gas content at 25 mol%. R-23 (CHF₃) was used as the HFC or PFC gas, but similar results could be achieved with other HFC gases such as R-32 (CH₂F₂), R-41 (CH₃F), R-134a (CF₃CH₂F), R-125 (CF₃CHF₂), and PFC gases such as CF₄, C₂F₆, and C₃F₈.

### 2. Gas chromatography analysis results

The results of gas chromatography analysis according to the comparative examples and examples are shown in Table 1 below. Table 1 lists the content of each of the sampled products in units of % by volume: intermediate [1,4-dibromo-2,3-dichlorohexafluorobutane (Br-CF₂-CFCl-CFCl-CF₂-Br)], isomer [1,4-dibromo-1,3-dichlorohexafluorobutane (Br-CF₂-CFCl-CF₂-CFCl-Br)], high boiler A [1,6-dibromo-2,3,5-trichlorononafluorohexane (Br-(CF₂-CFCl)₃-Br)], and high boiler B [1, 8-dibromo-2, 3, 5, 7-tetrachlorodecafluoroheptane (Br-(CF₂-CFCl)₄-Br)].

**[Table 1]**

| Classification | Number of operating lamps | Concentration of solution | Concentration of diluent gas | vol.% | | | |
|---|---|---|---|---|---|---|---|
| | | | | Intermediate | Isomer | High BP material A | High BP material B |
| Comparative Example 1 | 1 | CF₂BrCFClBr 100mol% | Nitrogen 0mol% | 0.1220 | 0.0085 | 0.1351 | 0.1301 |
| Comparative Example 2 | 1 | CF₂BrCFClBr 80mol | | 0.1130 | 0.0065 | 0.1051 | 0.1031 |
| | | MC 20 mol% | | | | | |
| Comparative Example 3 | 1 | CF₂BrCFClBr 65mol% | CTFE 100mo1% | 0.1020 | 0.0053 | 0.0551 | 0.0131 |
| | | MC 35 mol% | | | | | |
| Comparative Example 4 | 1 | CF₂BrCFClBr 20mol% | | 0.0720 | 0.0022 | 0.0151 | 0.0031 |
| | | MC 80 mol% | | | | | |
| Example 1 | 1 | CF₂BrCFClBr 65mol% | Nitrogen 50mol% | 0.1331 | 0.0104 | 0.0019 | 0.0011 |
| | | | CTFE 50mol% | | | | |
| Example 2 | 1 | | Nitrogen 75mol% | 0.1412 | 0.0110 | 0.0006 | 0.0005 |
| | | | CTFE 25mol% | | | | |
| Example 3 | 1 | | Nitrogen 90mol% | 0.1450 | 0.0113 | 0.0002 | 0.0001 |
| | | | CTFE 10mol% | | | | |
| Example 4 | 1 | | Nitrogen 98mol% | 0.1445 | 0.0112 | 0.0001 | 0.0001 |
| | | | CTFE 2mol% | | | | |
| Example 5 | 3 | | Nitrogen 90mol% | 0.4229 | 0.0338 | 0.0003 | 0.0002 |
| | | | CTFE 10mol% | | | | |
| Example 6 | 7 | | Nitrogen 90mol% | 1.0115 | 0.0787 | 0.0005 | 0.0003 |
| | | MC 35 mol% | CTFE 10mol% | | | | |
| Example 7 | 1 | | Nitrogen 0mol% | 0.1425 | 0.0083 | 0.0005 | 0.0003 |
| | | | HFC/PFC 75mol% | | | | |
| | | | CTFE 25mol% | | | | |
| Example 8 | 1 | | Nitrogen 50mol% | 0.1422 | 0.0087 | 0.0005 | 0.0003 |
| | | | HFC/PFC 25mol% | | | | |
| | | | CTFE 25mol% | | | | |
| Example 9 | 1 | | Nitrogen 25mol% | 0.1423 | 0.0081 | 0.0005 | 0.0003 |
| | | | HEC/PFC 50mol% | | | | |
| | | | CTFE 25mol% | | | | |
| Example 10 | 7 | | Nitrogen 50mol% | 1.0332 | 0.0609 | 0.0005 | 0.0003 |
| | | | HFC/PFC 25mol% | | | | |
| | | | CTFE 25mol% | | | | |

From Table 1 above, it can be seen that dilution with a solvent reduces the amount of high boiling point (BP) materials as compared to Example 1 in which the reaction was carried out without using a solvent in the presence of only CTFE without a diluent gas (Examples 2 to 4). The comparative examples show that the solvent (MC) inhibits the formation of the high BP materials at a solvent content of 35 mol% in the 1,2-dibromo-1-chlorotrifluoroethane solution and that at a solvent content of more than 35 mol%, the formation of the high BP materials can be reduced, but the formation of the intermediate is also reduced. Therefore, a solvent content of 35 mol% was the most effective, when taking into account selectivity and reactivity,

The variation of the diluent gas concentration was examined while maintaining the solvent content at 35 mol%, and it can be seen that the formation of high BP materials is significantly reduced when the diluent gas is added compared to Example 3 in which the reaction was conducted with CTFE alone without using a diluent gas. When Comparative Example 3 is compared with Example 1, the generation of the intermediate was increased, and the formation of high BP materials was significantly reduced by supplying 50 mol% of nitrogen gas (50 mol% of CTFE) as the diluent gas. In the case of Examples 2 through 4 in which the nitrogen gas was increased to 75 mol% or more (25 mol% of CTFE or less), the generation of the intermediate was found to be further increased and the generation of high BP materials was further suppressed.

This characteristic was also maintained in the case of Examples 5 and 6 in which the number of operating ramps was increased, and it was found that by increasing the number of operating ramps, the production of the intermediate increased proportionally to the number of the ramps.

In addition, it can be seen from Examples 7 through 10 that the formation of isomers is inhibited by the inclusion of HFC or PFC gas in the diluent gas. Examples 7 through 10 in which the CTFE content was fixed to 25 mol% and the HFC or PFC content in the diluent gas was varied in a range of 25 mol% to 75 mol% showed that the generation of isomers was somewhat suppressed compared to Examples 2 in which the CTFE content was set to 25 mol%. These characteristics were achieved in Example 10 in which the number of operating ramps was increased to seven.

While the foregoing has been described with reference to some embodiments and drawings, those are illustrative only, and it will be apparent to the ordinarily skilled in the art that various modifications are possible without departing from the scope of the present disclosure.

Therefore, the scope of protection of the present disclosure should be determined by the recitation of the patent claims and their equivalents.

## Claims

1. A method of preparing 1,4-dibromo-2,3-dichlorohexafluorobutane (Br-CF₂-CFCl-CFCl-CF₂-Br), the method comprising: reacting a 1,2-dibromo-1-chlorotrifluoroethane (Br-CF₂-CFCl-Br) solution diluted with a solvent with CTFE (CF₂=CFCl) mixed with a diluent gas by light (UV) initiation to produce 1,4-dibromo-2,3-dichlorohexafluorobutane.

2. The method of claim 1, wherein the solvent is methylene chloride (MC, CH₂Cl₂).

3. The method of claim 1, wherein the diluent gas comprises an inert gas.

4. The method of claim 1, wherein the diluent gas comprises hydrofluorocarbon (HFC) gas or perfluorocarbon (PFC) gas.

5. The method of claim 1, wherein the diluent gas comprises at least one inert gas and at least one gas selected from hydrofluorocarbon (HFC) gas and perfluorocarbon (PFC) gas.

6. The method of claim 1, wherein the 1,2-dibromo-1-chlorotrifluoroethane (Br-CF₂-CFCl-Br) solution has a solvent content of 90 mol% or less.

7. The method of claim 1, wherein a total gas comprising the diluent gas and a CTFE gas has a CTFE gas content of 50 mol% or less.

8. The method of claim 1, further comprising:
reacting CTFE (CF₂=CFCl) with bromine (Br₂) to produce 1,2-dibromo-1-chlorotrifluoroethane (Br-CF₂-CFCl-Br).

9. A method of preparing hexafluoro-1,3-butadiene (C₄F₆) using 1,4-dibromo-2,3-dichlorohexafluorobutane (Br-CF₂-CFCl-CFCl-CF₂-Br) as an intermediate, the method comprising: a photoreaction step of preparing 1,4-dibromo-2,3-dichlorohexafluorobutan by reacting a 1,2-dibromo-1-chlorotrifluoroethane (Br-CF₂-CFCl-Br) solution diluted with a solvent with a CTFE (CF₂=CFCl) gas mixed with a diluent gas by light (UV) initiation; and a dehalogenation step of removing halogens except fluorine from the prepared 1,4-dibromo-2,3-dichlorohexafluorobutane.

10. The method of claim 9, wherein the dehalogenation step is carried out in the presence of zinc (Zn) and isopropyl alcohol.

11. The method of claim 9, further comprising preparing 1,2-dibromo-1-chlorotrifluoroethane (Br-CF₂-CFCl-Br) by reacting CTFE (CF₂=CFCl) with bromine (Br₂) before the photoreaction step.
